# EUROPÄISCHE PATENTSCHRIFT

(11) **EP 3 388 146 B1**
(45) Veröffentlichungstag und Bekanntmachung des Hinweises auf die Patenterteilung: **28.08.2019**
(21) Anmeldenummer: 17165989.9
(22) Anmeldetag: 11.04.2017
(51) Int. Cl.: B01J 23/46, C07F 9/50, C07F 9/6571, C07F 15/06, C07C 45/50, C07C 47/02

(54) **LIGANDEN UND VERFAHREN ZUR HYDROFORMYLIERUNG UNTER EINSATZ EINES 5-METHYL-4,4-DIPHENYL-1,3,2-DIOXAPHOSPHOLANS**
LIGANDS AND METHOD FOR HYDROFORMYLATION USING A 5-METHYL-4,4-DIPHENYL-1,3,2-DIOXAPHOSPHOLANE
LIGANDS ET PROCÉDÉ D'HYDROFORMYLATION À L'AIDE D'UN 5-MÉTHYL-4,4-DIPHÉNYL-1,3,2-DIOXAPHOSPHOLANE

(43) Veröffentlichungstag der Anmeldung: 17.10.2018
(73) Patentinhaber: Evonik Degussa GmbH, 45128 Essen (DE)
(72) Erfinder: BÖRNER, Armin, 18059 Rostock (DE); FRANKE, Robert, 45772 Marl (DE); SELENT, Detlef, 18059 Rostock (DE)

(56) Entgegenhaltungen:
- EP-A1- 3 029 013
- EP-A1- 3 029 047
- EP-A1- 3 147 288
- US-A- 5 043 465
- US-A- 5 260 491
- ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803

## Beschreibung

Die Erfindung betrifft Liganden und ein Verfahren zur Hydroformylierung unter Einsatz eines 5-Methyl-4,4-diphenyl-1,3,2-dioxaphospholans.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden. Liganden in Hydroformylierungsreaktionen sind beispielsweise in EP 3 029 047 A1, EP 3 029 013 A1, EP 3 147 288 A1 und US 5 260 491 offenbart.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, ein Verfahren zur Hydroformylierung bereitzustellen, welches durch einen Ligand-Metall-Komplex katalysiert wird. Ausgangsstoff für die Ligandensynthese sollte hierbei (-)-1,1-Diphenylpropan-1,2-diol sein.

Die Aufgabe wird gelöst durch eine Verbindung nach Anspruch 1, beziehungsweise durch ein Verfahren nach Anspruch 6.

Verbindung gemäß einer der folgenden Strukturen:

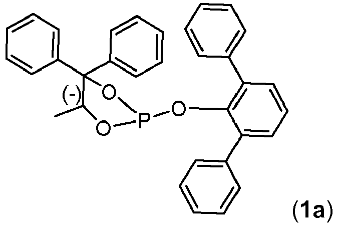

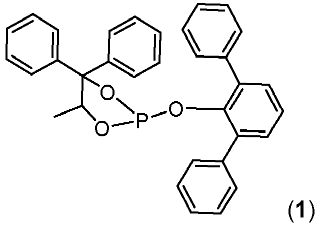

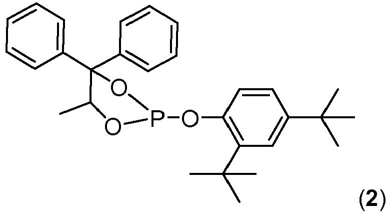

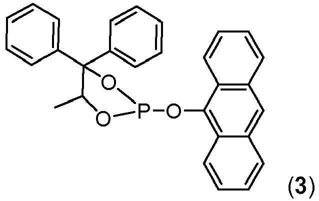

Verbindung, welche die folgende Struktur aufweist:

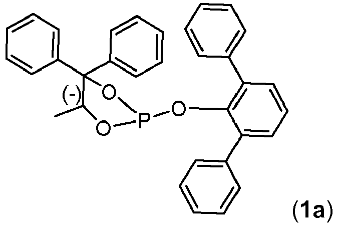

Verbindung, welche die folgende Struktur aufweist:

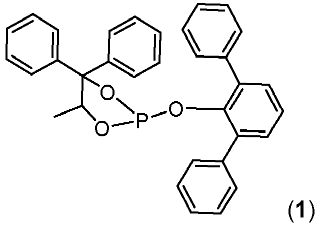

Verbindung, welche die folgende Struktur aufweist:

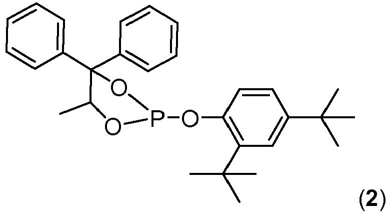

Verbindung, welche die folgende Struktur aufweist:

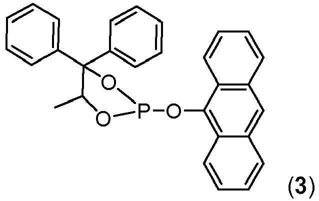

Verfahren zur Hydroformylierung umfassend die Verfahrensschritte:
a) Vorlegen eine Olefins;
b) Zugabe eines Komplexes umfassend:
   - ein Metallatom ausgewählt aus: Rh, Ru, Co, Ir, und
   - einen Liganden ausgewählt aus: 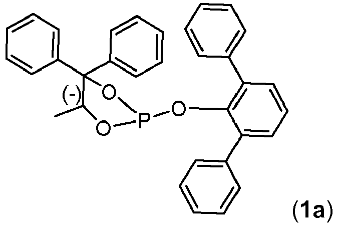 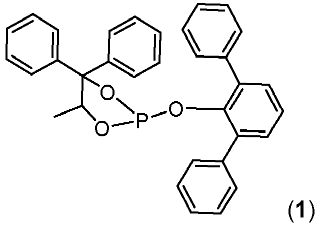 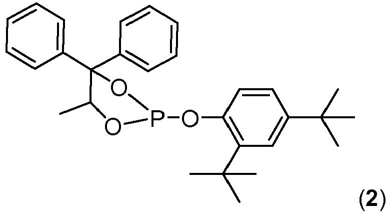 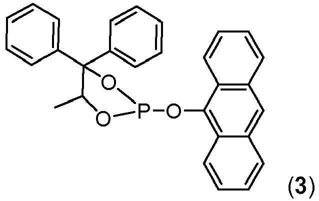 oder
      Zugabe einer Komplexvorstufe umfassend ein Metallatom ausgewählt aus: Rh, Ru, Co, Ir, und einer Verbindung ausgewählt aus: 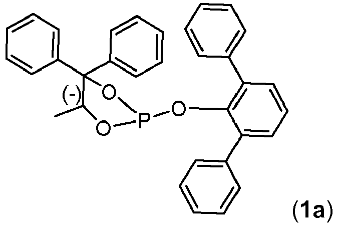 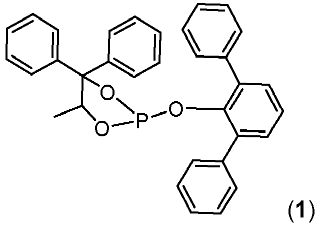 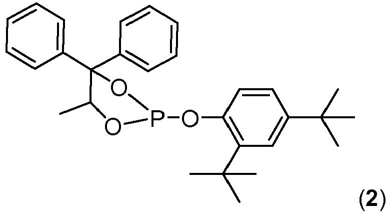 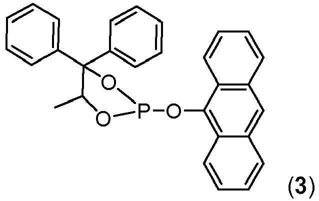
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer Variante des Verfahrens ist das Metallatom Rh.

In einer Variante des Verfahrens umfasst die Komplexvorstufe Cyclooctadien.

In einer Variante des Verfahrens handelt es sich bei der Komplexvorstufe um [(acac)Rh(COD)]. Hierbei stehen "acac" für Acetylacetonat-Anion und "COD" für Cyclooctadien.

In einer Variante des Verfahrens weist der Ligand und die Verbindung die folgende Struktur auf:

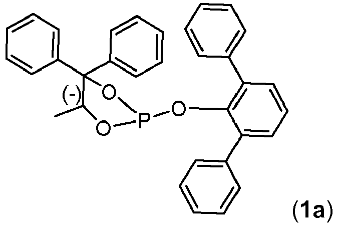

In einer Variante des Verfahrens weist der Ligand und die Verbindung die folgende Struktur auf:

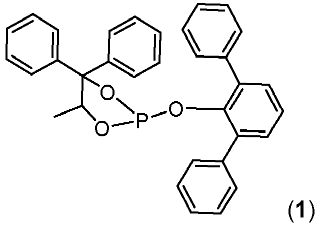

In einer Variante des Verfahrens weist der Ligand und die Verbindung die folgende Struktur auf:

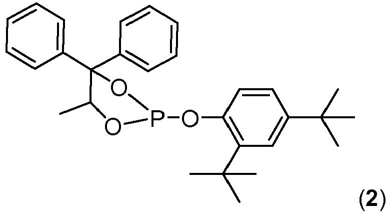

In einer Variante des Verfahrens weist der Ligand und die Verbindung die folgende Struktur auf:

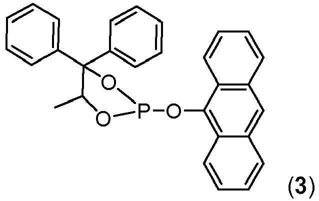

In einer Variante des Verfahrens wird das Reaktionsgemisch im Verfahrensschritt d) auf eine Temperatur im Bereich von 100 °C bis 140 °C erwärmt.

Im Folgenden wird die Erfindung anhand von Ausführungsbeispielen näher erläutert.

### Analytik

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl₃ verwendet. Die ¹H- und ¹³C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Synthesen

Als Edukt für die Synthese wurde (-)-1,1-Diphenylpropan-1,2-diol verwendet, welches von Aldrich bezogen wurde:

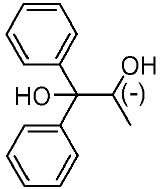

### a) 2-Chlor-5-methyl-4,4-diphenyl-1,3,2-dioxaphospholan (Vorstufe)

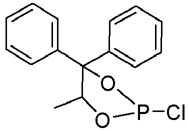

Eine bei -40 °C gerührte Lösung von (*S*)-(-)-1,1-Diphenylpropan-1,2-diol (0,998 g; 4,373 mmol) in THF (30 ml) wird innerhalb von 30 min tropfenweise mit einer Lösung von PCl₃ (0,961 g; 6,997 mmol) in THF (9 ml) und anschließend mit einer Lösung von Triethylamin (1,327 g; 13,119 mmol) in THF (5 ml) versetzt. Man lässt auf Raumtemperatur kommen, rührt über Nacht und filtriert. Das Filtrat wird im Vakuum zur Trockne eingeengt und der erhaltene Rückstand mit Toluol (8 ml) verrührt. Filtration, Einengen des Filtrates im Vakuum und Trocknen des Rückstandes bei 0,1 mbar für 4 h ergibt 1,009 g (3,477 mmol; 79%). ³¹P-NMR (CD₂Cl₂): 8 165,3 (s) ppm.
Die Substanz hält 95% der Signalintensität des ³¹P-NMR-Spektrums und wurde wie erhalten in den nachfolgenden Schritt b) bis g) eingesetzt.

### b) 2-([1,1':3',1"-Terphenyl]-2'-yloxy)-5-methyl-4,4-diphenyl-1,3,2-dioxaphospholan (Ligand 1) und das enantiomerenreine R,S-Diastereomer (Ligand 1a)

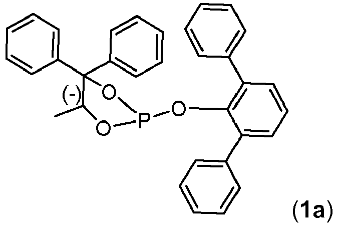

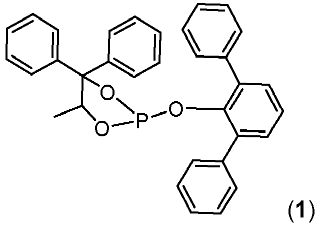

Eine bei 0 °C gerührte Lösung von 2,6-Diphenylphenol (0,442 g; 1,796 mmol) und Triethylamin (1,665 g; 16,450 mmol) in Toluol (7 ml) wird tropfenweise mit einer Lösung von 2-Chlor-5-methyl-4,4-diphenyl-1,3,2-dioxaphospholan (0,553 g; 1,890 mmol) in Toluol (4 ml) versetzt. Man lässt auf Raumtemperatur kommen, rührt über Nacht und filtriert. Das Filtrat wird eingeengt und der erhaltene Rückstand im Vakuum getrocknet. Ausbeute: 0,780 g (1,552 mmol; 86%).
³¹P-NMR (CD₂Cl₂): δ 131,7 (s); 148,1 (s) ppm.

Das *R,S*-Diastereomer (**1a**) kann aus einer Lösung des so erhaltenen Isomerengemisches in Toluol durch Fällen mit Heptan erhalten werden. Seine Konfiguration wurde durch Einkristallröntgenstrukturanalyse bestätigt.

Elementaranalyse *R,S*-Diastereomer (ber. für C₃₃H₂₇O₃P= 502,52 g/mol): C 78,99 (78,87); H 5,33 (5,42); P 6,09 (6,16) %.
³¹P-NMR (CD₂Cl₂): δ = 148,1 (s) ppm.
¹H-NMR (CD₂Cl₂): δ = 0,97 (d, 3H, ³*J*_{HH}= 6,6 Hz); 5,08 (dq, 1H, ³*J*_{HH}= 6,6 Hz; *J*_{HP}= 16,8 Hz), 7,11-7,63 (m, 23H) ppm.
¹³C NMR (CD₂Cl₂) δ = 146,8 (d; *J*_{CP}= 7,5 Hz); 144,7; 141,1; 138,7; 136,1; 136,0; 130,5; 130,4; 128,1; 127,9; 127,6; 127.3; 127,0; 126,2; 125,6; 124,2; 90,4 (d, *J*_{CP}= 9,0 Hz); 78,8 (d, *J*_{CP}= 7,5 Hz); 19,71 ppm.
ESI-TOF/HRMS: *m*/*e* 503,17732 (M+H)⁺.

### c) 2-(2,4-Di-tert-butylphenoxy)-5-methyl-4,4-diphenyl-1,3,2-dioxaphospholan (Ligand 2)

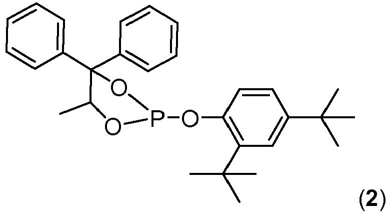

Zur einer bei 0 °C gerührten Lösung von 2,4-Di-*tert*-butylphenol (0,439 g; 2,128 mmol) und Triethylamin (2,7 ml) in Toluol (8 ml) wird tropfenweise eine Lösung von 2-Chlor-5-methyl-4,4-diphenyl-1,3,2-dioxaphospholan (0,654 g; 2,237 mmol, siehe Stufe a) bei der Synthese von Ligand **1**) in Toluol (5 ml) gegeben. Man erwärmt auf Raumtemperatur, rührt über Nacht, filtriert und entfernt das Lösungsmittel und überschüssiges Amin im Vakuum. Der viskose Rückstand wird 2 h bei 50°C /0,1 mbar getrocknet und dann in warmem Acetonitril (4 ml) gelöst. Lagerung bei -29°C ergibt einen mikrokristallinen Niederschlag. Ausbeute: 0,697 g (1,507 mmol; 70%).

Elementaranalyse (ber. für C₂₉H₃₅O₃P= 462,57 g/mol): C 75,59 (75,30); H 7,93 (7,63); P 6,65 (6,70) %.
³¹P-NMR (CD₂Cl₂): δ = 129,5 (s); 141,6 (s) ppm.
¹H-NMR (CD₂Cl₂): δ = 1,22 (s, 9H); 1,28 (d, ³*J*_{HH}= 6,4 Hz, 3H); 1,34 (s, 9H), 5,36 (q, ³*J*_{HH}= 6,4 Hz, 1H), 7,04-7,59 (m, 13H) ppm.
¹³C-NMR (CD₂Cl₂) δ = 19,0 (d, *J*_{CP}= 5.7 Hz) 29.9, 31.7, 34.8, 35.0, 79.1 (d, *J*_{CP}= 6.1 Hz), 90.8 (d, *J*_{CP}= 7.9 Hz), 119.5 (d, *J*_{CP}= 17.2 Hz), 123.9, 124.7, 127.1, 127.9, 128.0, 128.2, 128.6, 139.7, 141.4 (d, *J*_{CP}= 3.0 Hz), 143.3, 146.2, 149.0 (d, *J*_{CP}= 7.8 Hz) ppm.
ESI-TOF/HRMS: *m*/*e* 463,24014 (M+H)⁺.

### d) 2-(Anthracen-9-yloxy)-5-methyl-4,4-diphenyl-1,3,2-dioxaphospholan (Ligand 3)

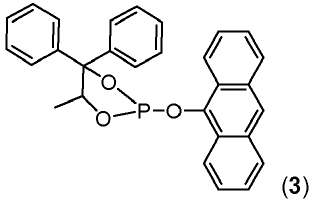

Zur einer bei 0 °C gerührten Lösung von Anthron (0,332 g; 1,709 mmol) und Triethylamin (2,2 ml) in Toluol (7 ml) wird tropfenweise eine Lösung von 2-Chlor-5-methyl-4,4-diphenyl-1,3,2-dioxaphospholan (0,525 g; 1,794 mmol, siehe Stufe a) bei der Synthese von Ligand **1**) in Toluol (5 ml) gegeben. Man erwärmt auf Raumtemperatur, rührt über Nacht, filtriert und entfernt das Lösungsmittel und überschüssiges Amin im Vakuum. Der viskose Rückstand wird 1 h bei 40°C/0,1 mbar getrocknet und dann erneut in Toluol (5 ml) gelöst. Nach Filtration wird das Filtrat im Vakuum eingeengt und der Rückstand 5h bei 40°C/0,1 mbar getrocknet. Ausbeute: 0,71 g (1,576 mmol; 92%). Lagerung im Kühlschrank erforderlich.

Elementaranalyse (ber. für C₂₉H₂₃O₃P= 450,47 g/mol): C 77,44 (77,32); H 5,29 (5,15); P 6,77 (6,88) %.
³¹P-NMR (CD₂Cl₂): δ = 133,9 (s); 148,7 (s) ppm.
¹H-NMR (CD₂Cl₂): δ = 1,31/1,51 (2d, ³*J*_{HH}= 6,3/6,7 Hz; 3H); 5,47/5,64 (q/dq, ³*J*_{HH}= 6,3/6,7 Hz, ³*J*_{HP}= 0/6,7 Hz, 1H); 1,34 (s, 9H), 5,36 (q, ³*J*_{HH}= 6,4 Hz, 1H), 7,04-7,59 (m, 13H) ppm.
¹³C-NMR (CD₂Cl₂) δ = 14.4, 19.1, 19.2, 21.1, 21.6, 23.2, 32.4, 79.2, 79.3, 80.5, 80.6, 91.7, 91.8, 92.2, 122.8, 123.0, 123.3, 123.4, 123.4, 124.9, 124.9, 125.3, 125.3, 125.4, 125.7, 125.9, 126.0, 126.2, 126.3, 126.5, 126.7, 127.0, 127.1, 127.4, 127.9, 128.0, 128.2, 128.3, 128.4, 128.6, 128.6, 128.7, 128.7, 128.8, 128.9, 129.2, 129.4, 132.5, 132.6, 138.4, 141.2, 141.2, 141.5, 142.8, 144.3, 145.0 ppm.
ESI-TOF/HRMS: *m*/*e* 451,14656 (M+H)⁺.

### e) 2,2'-Bis((5-methyl-4,4-diphenyl-1,3,2-dioxaphospholan-2-yl)oxy)-1,1'-biphenyl (Ligand 4)

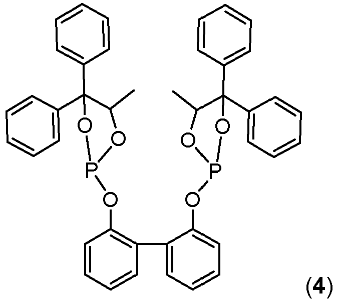

Zur einer bei 0 °C gerührten Lösung von 2,2'-Dihydroxybiphenyl (0,362 g; 1,941 mmol) und Triethylamin (0,84 ml) in Toluol (8 ml) wird tropfenweise eine Lösung von 2-Chlor-5-methyl-4,4-diphenyl-1,3,2-dioxaphospholan (1,193 g; 4,076 mmol, siehe Stufe a) bei der Synthese von Ligand **1**) in Toluol (21 ml) gegeben. Die Mischung wird über Nacht bei Raumtemperatur und 2 h bei 70°C gerührt. Es wird durch Kieselgel filtriert und das Filtrat im Vakuum zur Trockne eingeengt. Der viskose Rückstand wird 3 h bei 50°C/0,1 mbar getrocknet. Ausbeute: 1,16 g (1,66 mmol; 86%). Zur Reinigung erfolgte Fällung aus einer Lösung in Heptan (14 ml) bei -29 °C.

Elementaranalyse (ber. für C₄₂H₃₆O₆P₂= 450,47 g/mol): C 72,26 (72,20); H 5,20 (5,19); P 8,67 (8,87) %.
³¹P-NMR (CD₂Cl₂): δ= 130,4 (d, *J*_{PP}= 4,5 Hz); 130,5 (s); 141,9 (d, *J*_{PP}= 4,5 Hz) 142,4 (s) ppm. ¹H-NMR (CD₂Cl₂): δ = 0,87/1,04(d, ³*J*_{HH}= 6,7 Hz/dd, ³*J*_{HH}= 6,3 Hz; *J*_{HP}= 3,3Hz. 6H); 4,74/5,25 (q, ³*J*_{HH}= 6,7 Hz/dq ³*J*_{HH}= 6,3 Hz; *J*_{HP}= 2,5 Hz. 2H); 6,99-7,54 (m, 28H) ppm.
ESI-TOF/HRMS: *m*/*e* 737,16356 (M+K)⁺.

### f) 2,2'-Bis((5-methyl-4,4-diphenyl-1,3,2-dioxaphospholan-2-yl)oxy)-1,1'-binaphthalin (Ligand 5)

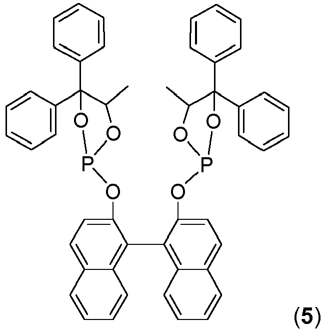

Zur einer bei 0 °C gerührten Lösung von 2-Chlor-5-methyl-4,4-diphenyl-1,3,2-dioxaphospholan (1,244 g; 4,251 mmol, siehe Stufe a) bei der Synthese von Ligand **1**) in Toluol (20 ml) wird eine Lösung von (-)-2,2'-Dihydroxybinaphthyl (0,580 g; 2,025 mmol) und Triethylamin (1,2 ml) in Toluol (8 ml) tropfenweise zugegeben. Die Mischung wird 3d bei Raumtemperatur gerührt, es wird filtriert und das Filtrat im Vakuum zur Trockne eingeengt. Der erhaltene Rückstand wird in Toluol (12 ml) gelöst, die Lösung filtriert und bei Raumtemperatur mit Heptan (9 ml) versetzt. Stehenlassen über Nacht bedingt Abscheidung eines weißen Feststoffs, welcher abgetrennt wird. Das Filtrat wird zur Trockne eingeengt, und der Rückstand im Vakuum (0,1 mbar) getrocknet. Ausbeute: 0,776 g (0,972 mmol; 48%)

Elementaranalyse (ber. für C₅₀H₄₀O₆P₂= 798,79 g/mol): C 75,28 (75,18); H 5,36 (5,05); P 7,48 (7,76) %.
³¹P-NMR (CD₂Cl₂): δ = 131,1 (d, *J*_{PP}= 7,7 Hz); 131,6 (s); 141,9 (s); 142,1 (d, *J*_{PP}= 7,7 Hz) ppm. ¹H-NMR (CD₂Cl₂): δ = 0,56 (m, 4H); 0.74 (m, 2H); 4,28/5,13 (m/m, 2H); 6,97-7,78 (m, 28H) ; 7,99-8,16 (m, 4H) ppm.
¹³C-NMR (CD₂Cl₂): δ = 18.2, 18.3, 20.0, 20.1, 20.5, 21.9, 78.3, 78.4, 78.5, 80.1, 80.2, 80.2, 81.5, 90.5, 90.6, 90.7, 90.7, 91.4, 91.4, 91.5, 92.3, 115.3, 118.4, 121.5, 121.6, 121.6, 121.8, 122.0, 122.1, 123.6, 123.7, 123.8, 124.0, 124.1, 124.1, 125.3, 125.3, 125.6, 125.9, 126.1, 126.5, 126.5, 126.6, 126.9, 126.9, 127.0, 127.1, 127.2, 127.3, 127.6, 128.1, 128.2, 128.2, 128.3, 128.4, 128.6, 128.6, 128.8, 128.8, 129.0, 129.0, 129.3, 129.4, 129.6, 130.2, 130.3, 130.3, 130.4, 130.7, 131.2, 131.2, 131.3, 131.4, 134.7, 134.8, 134.8, 138.5, 139.9, 141.2, 141.2, 141.3, 141.9, 142.7, 142.8, 145.2, 145.3, 148.6, 148.7, 148.7, 148.8, 149.4, 149.5 ppm.
ESI-TOF/HRMS: *m*/*e* 799,23708 (M+H)⁺.

### g) 2,2'-((3,3'-Di-tert-butyl-5,5'-dimethoxy-[1,1'-biphenyl]-2,2'-diyl)bis(oxy))bis(5-methyl-4,4-diphenyl-1,3,2-dioxaphospholan) (Ligand 6)

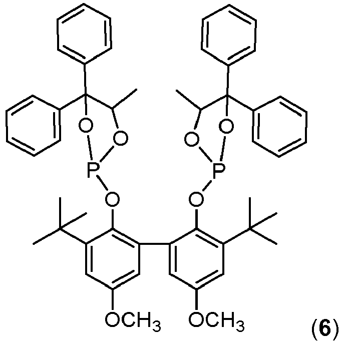

Eine Lösung von 3,3'-Di-tert-butyl-5,5'-dimethoxy-[1,1'-biphenyl]-2,2'-diol (0,999 g; 2,786 mmol) in THF (10 ml) wird bei -20°C mit einer 0,53 M Lösung von n-Butyllithium in Hexan (10,5 ml; 5,572 mmol) versetzt. Man lässt noch 20 min in der Kälte rühren, erwärmt dann auf Raumtemperatur und addiert tropfenweise eine Lösung von 2-Chlor-5-methyl-4,4-diphenyl-1,3,2-dioxaphospholan (1,688 g; 5,768 mmol, siehe Stufe a) bei der Synthese von Ligand **1**). Die Reaktionsmischung wird über Nacht gerührt, über eine mit Kielgel beschichtete Fritte filtriert und das erhaltene Filtrat zur Trockne eingeengt. Der erhaltene weiße Feststoff wird 2 h bei 50°C/0,1 mbar getrocknet und säulenchromatografisch (Hexan/Ethylazetat = 8:1, R*_{f}*= 0,30) gereinigt. Ausbeute: 1,036 g (1,19 mmol; 43%).

Elementaranalyse (ber. für C₅₂H5₆O₈P₂= 870,95 g/mol): C 71,65 (71,71); H 6,75 (6,48); P 7,05 (7,11) %.
³¹P-NMR (CD₂Cl₂) δ = 130,4 (s); 131,1 (s); 131,2 (s); 133,1 (s); 133,9 (d, *J*_{PP}=5,8Hz); 146,0 (s); 146,7 (s); 148,0 (d, *J*_{PP}=5,8Hz); 148,4 (s) ppm.
¹H-NMR (CD₂Cl₂): δ = 0,87-1,14(m, 6H); 1,25-1,33 (überlappende Singuletts, 9H); 1,53-1,60 (überlappende Singuletts, 9H); 3,76-3,80 (überlappende Singuletts, 6H); 4,86-5,21 (m, 2H); 6,60-7,48 (m, 24H) ppm.
¹³C-NMR (CD₂Cl₂) δ = 14.3, 18.3, 18.8, 20.3, 23.1, 27.3, 30.5, 30.6, 30.8, 32.0, 35.3, 35.4, 35.6, 55.7, 55.8, 55.8, 55.9, 77.9, 79.1, 90.6, 114.4, 114.7, 114.8, 115.4, 125.9, 125.9, 126.2, 126.5, 126.7, 127.4, 127.5, 127.7, 127.9, 127.9, 128.2, 128.3, 128.4, 128.5, 128.6, 128.6, 129.0, 129.4, 133.8, 141.7, 142.2, 143.3, 143.5, 143.8, 145.5, 154.9, 155.0 ppm.
ESI-TOF/HRMS: *m*/*e* 871,35139 (M+H)⁺.

### Durchführung der Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System und unter Argon aufbewahrt. Das als Substrat eingesetzte *n*-Octen (Oxeno GmbH) wurden über Natrium am Rückfluß erhitzt und unter Argon destilliert.

Für die Versuche mit *n*-Octen wurden im Autoklaven unter Argonatmosphäre Lösungen der Katalysatorvorstufe und des Liganden gemischt. Als Katalysatorvorstufe kam [(acac)Rh(COD)] (Umicore, acac=Acetylacetonat-Anion; COD=1,5-Cyclooctadien) zum Einsatz. Für Versuche mit n-Octen wurden 10 ml einer 4,31 millimolaren Lösung in den Autoklaven gegeben. Anschließend wurde die entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurden n-Octen (10,70 g; 95,35 mmol) eingefüllt. Der Autoklav wurde bei einem Gesamtgasdruck (Synthesegas: Linde; H₂ : CO = 1:1) von a) 42 bar für einen Enddruck von 50 bar, b) 12 bar für den Enddruck von 20 bar unter Rühren (1500 U/min) aufgeheizt. Nach Erreichen der Reaktionstemperatur von 120 °C wurde der Synthesegasdruck auf a) 48,5 bar für einen Enddruck von 50 bar, b) 19,5 bar für einen Enddruck von 20 bar erhöht und das Olefin mit einem in der Druckpipette eingestellten Überdruck von ca. 3 bar in den Autoklaven gepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

### Ergebnisse der Katalyseversuche

**Tabelle 1: Hydroformylierung n-Octen bei 20 bar**

| **Ligand** | **L/Rh** | **Gesamtausbeute Aldehyd (%)** |
|---|---|---|
| **1a*** | 5 | 99 |
| **1*** | 5 | 99 |
| **2*** | 5 | 99 |
| **3*** | 5 | 99 |
| **4** | 2 | 80 |
| **5** | 2 | 78 |
| **6** | 2 | 49 |

| | | |
|---|---|---|
| * erfindungsgemäßes Verfahren | | |

Reaktionsbedingungen: *T* = 120 °C, *p* = 20 bar CO/H₂, *t* = 4h, [Rh] = 100 ppm-m, Lösungsmittel: Toluol.

**Tabelle 2: Hydroformylierung n-Octen bei 50 bar**

| **Ligand** | **L/Rh** | **Gesamtausbeute Aldehyd (%)** |
|---|---|---|
| **1*** | 5 | 99 |
| **2*** | 5 | 99 |
| **3*** | 5 | 99 |
| **4** | 2 | 71 |
| **5** | 2 | 68 |

| | | |
|---|---|---|
| * erfindungsgemäßes Verfahren | | |

Reaktionsbedingungen: *T* = 120°C, *p* = 50 bar CO/H₂, *t* = 4h, [Rh] = 100 ppm-m, Lösungsmittel: Toluol.

Wie die Versuchsergebnisse zeigen, wird die Aufgabe durch das erfindungsgemäße Verfahren gelöst.

## Patentansprüche

1. Verbindung gemäß einer der folgenden Strukturen:

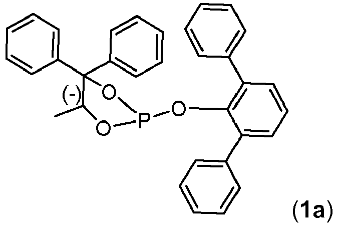

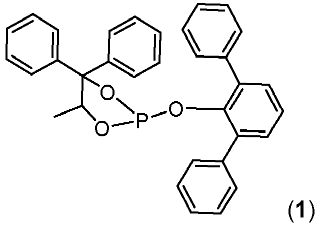

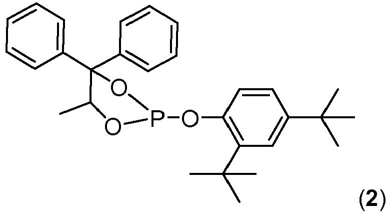

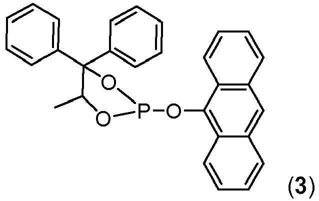

2. Verbindung nach Anspruch 1,
welche die folgende Struktur aufweist:

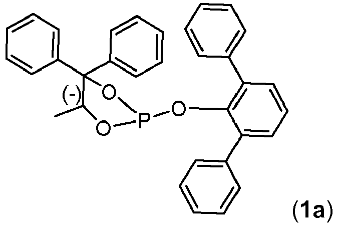

3. Verbindung nach Anspruch 1,
welche die folgende Struktur aufweist:

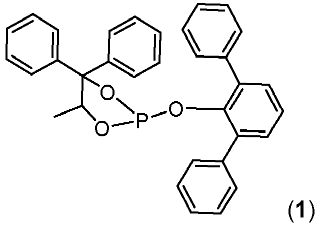

4. Verbindung nach Anspruch 1,
welche die folgende Struktur aufweist:

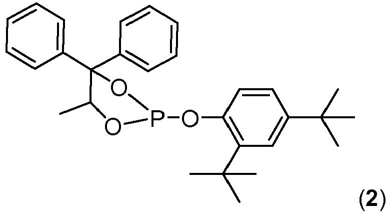

5. Verbindung nach Anspruch 1,
welche die folgende Struktur aufweist:

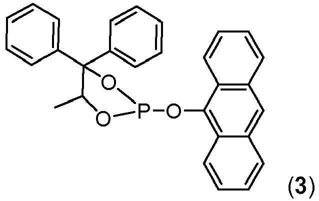

6. Verfahren zur Hydroformylierung umfassend die Verfahrensschritte:
a) Vorlegen eine Olefins;
b) Zugabe eines Komplexes umfassend:
- ein Metallatom ausgewählt aus: Rh, Ru, Co, Ir, und
- einen Liganden ausgewählt aus: 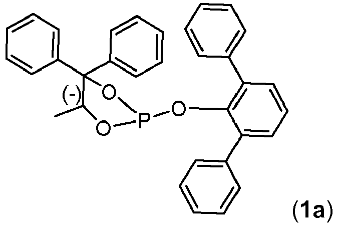 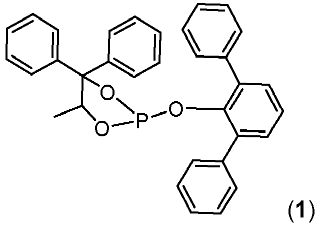 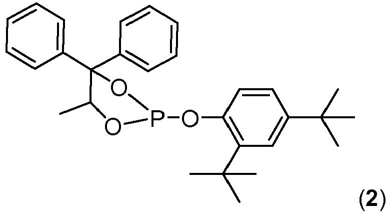 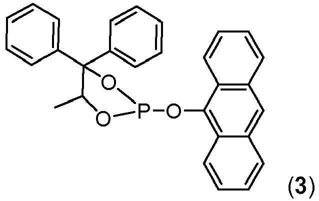 oder
Zugabe einer Komplexvorstufe umfassend ein Metallatom ausgewählt aus: Rh, Ru, Co, Ir, und einer Verbindung ausgewählt aus: 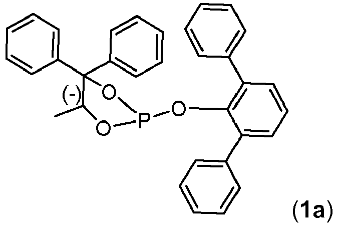 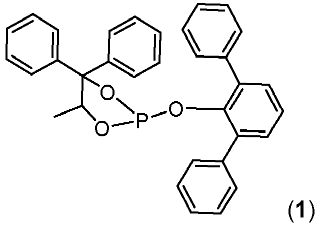 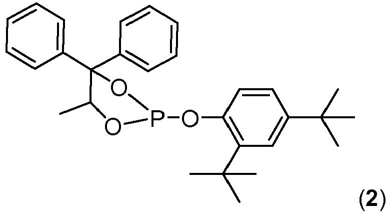 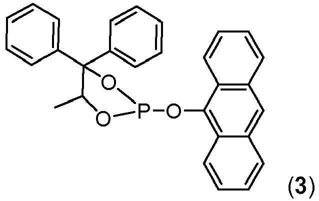
c) Zuführen von H₂ und CO,
d) Erwärmen des Reaktionsgemisches, wobei das Olefin zu einem Aldehyd umgesetzt wird.

7. Verfahren nach Anspruch 6,
wobei das Metallatom Rh ist.

8. Verfahren nach einem der Ansprüche 6 oder 7,
wobei die Komplexvorstufe Cyclooctadien umfasst.

9. Verfahren nach einem der Ansprüche 6 bis 8,
wobei es sich bei der Komplexvorstufe um [(acac)Rh(COD)] handelt.

10. Verfahren nach einem der Ansprüche 6 bis 9,
wobei der Ligand und die Verbindung die folgende Struktur aufweisen:

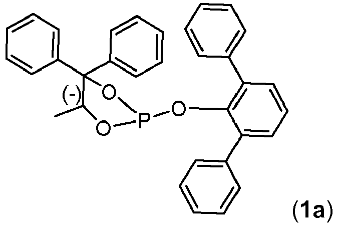

11. Verfahren nach einem der Ansprüche 6 bis 9,
wobei der Ligand und die Verbindung die folgende Struktur aufweisen:

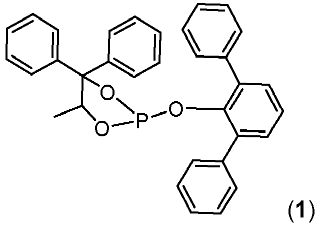

12. Verfahren nach einem der Ansprüche 6 bis 9,
wobei der Ligand und die Verbindung die folgende Struktur aufweisen:

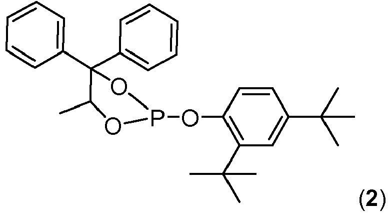

13. Verfahren nach einem der Ansprüche 6 bis 9,
wobei der Ligand und die Verbindung die folgende Struktur aufweisen:

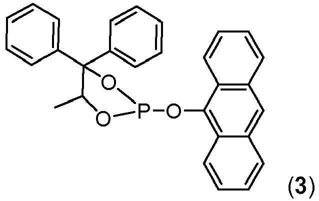

14. Verfahren nach einem der Ansprüche 6 bis 13,
wobei das Reaktionsgemisch im Verfahrensschritt d) auf eine Temperatur im Bereich von 100 °C bis 140 °C erwärmt wird.

## Claims

1. Compound according to one of the following structures:

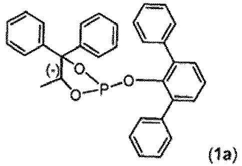

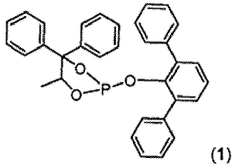

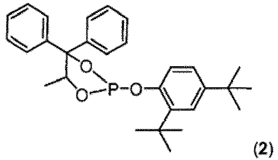

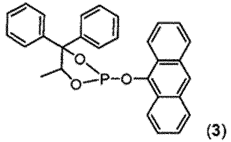

2. Compound according to Claim 1, which has the following structure:

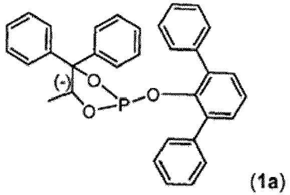

3. Compound according to Claim 1, which has the following structure:

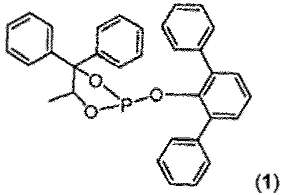

4. Compound according to Claim 1, which has the following structure:

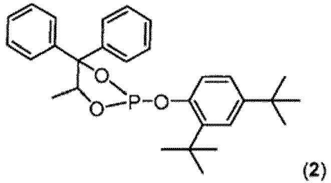

5. Compound according to Claim 1, which has the following structure:

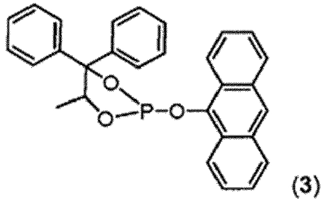

6. Method for hydroformylation comprising the method steps of:
a) initially charging an olefin;
b) adding a complex comprising:
- a metal atom selected from: Rh, Ru, Co, Ir and
- a ligand selected from: 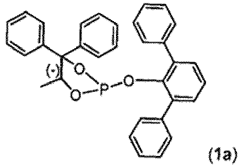 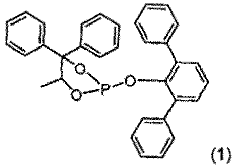 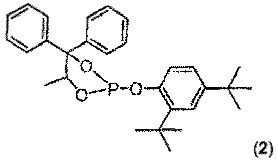 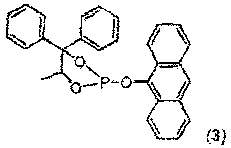 or
adding a complex precursor comprising a metal atom selected from: Rh, Ru, Co, Ir and a compound selected from: 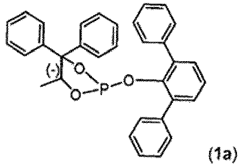 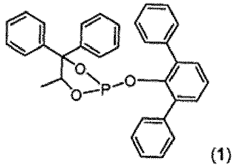 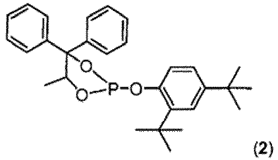 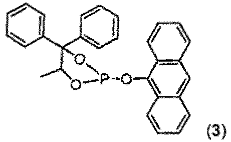
c) feeding in H₂ and CO,
d) heating the reaction mixture, whereupon the olefin is converted to an aldehyde.

7. Method according to Claim 6, wherein the metal atom is Rh.

8. Method according to either of Claims 6 or 7, wherein the complex precursor comprises cyclooctadiene.

9. Method according to any of Claims 6 to 8, wherein the complex precursor is [(acac)Rh(COD)].

10. Method according to any of Claims 6 to 9, wherein the ligand and the compound have the following structure:

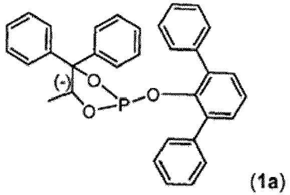

11. Method according to any of Claims 6 to 9, wherein the ligand and the compound have the following structure:

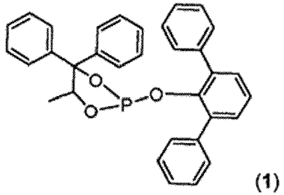

12. Method according to any of Claims 6 to 9, wherein the ligand and the compound have the following structure:

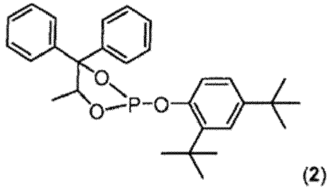

13. Method according to any of Claims 6 to 9, wherein the ligand and the compound have the following structure:

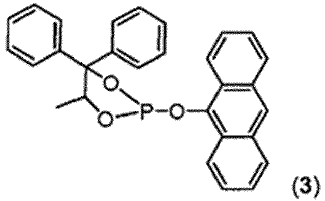

14. Method according to any of Claims 6 to 13, wherein the reaction mixture in method step d) is heated to a temperature in the range of 100°C to 140°C.

## Revendications

1. Composé selon l'une quelconque des structures suivantes :

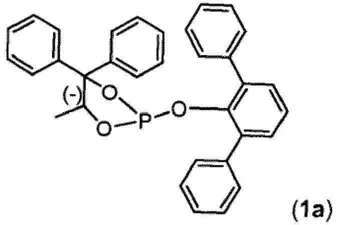

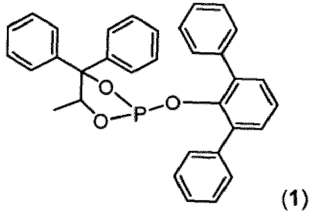

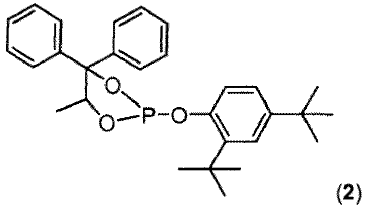

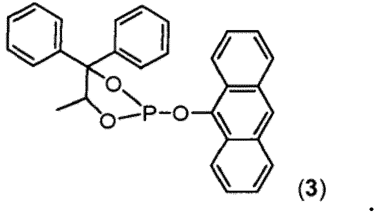

2. Composé selon la revendication 1, qui présente la structure suivante :

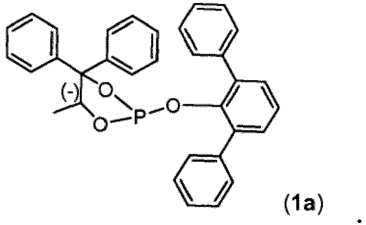

3. Composé selon la revendication 1, qui présente la structure suivante :

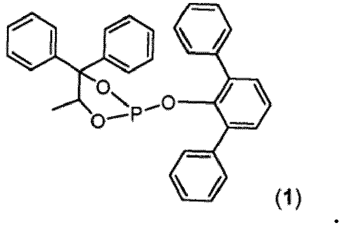

4. Composé selon la revendication 1, qui présente la structure suivante :

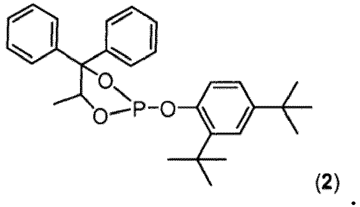

5. Composé selon la revendication 1, qui présente la structure suivante :

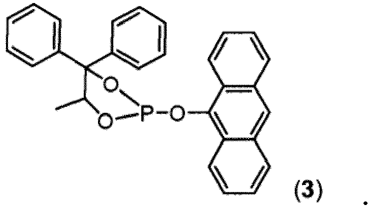

6. Procédé d'hydroformylation comprenant les étapes de procédé suivantes :
a) le chargement d'une oléfine ;
b) l'ajout d'un complexe comprenant :
- un atome métallique choisi parmi : Rh, Ru, Co, Ir, et
- un ligand choisi parmi : 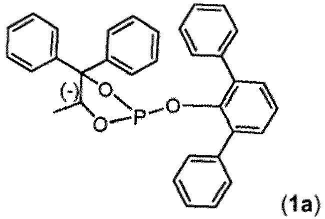 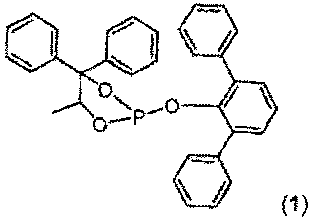 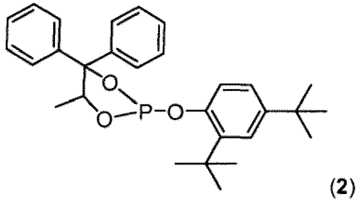 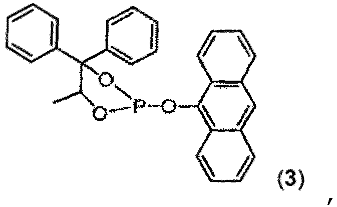 ou
l'ajout d'un précurseur de complexe comprenant un atome métallique choisi parmi : Rh, Ru, Co, Ir, et un composé choisi parmi : 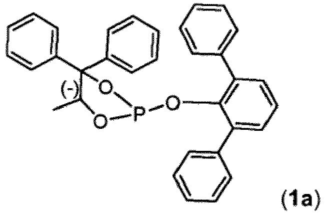 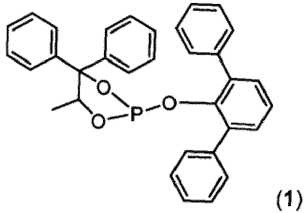 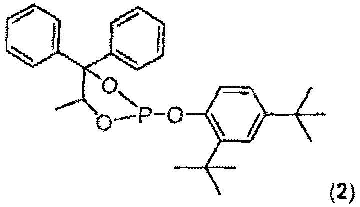 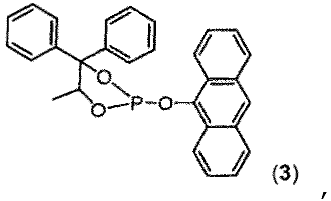
c) l'introduction d'H₂ et de CO,
d) le chauffage du mélange réactionnel, l'oléfine étant transformée en un aldéhyde.

7. Procédé selon la revendication 6, dans lequel l'atome métallique est Rh.

8. Procédé selon l'une quelconque des revendications 6 ou 7, dans lequel le précurseur de complexe comprend du cyclooctadiène.

9. Procédé selon l'une quelconque des revendications 6 à 8, dans lequel le précurseur de complexe est [(acac)Rh(COD)].

10. Procédé selon l'une quelconque des revendications 6 à 9, dans lequel le ligand et le composé présentent la structure suivante :

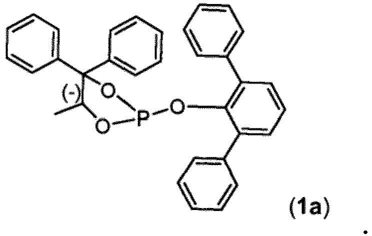

11. Procédé selon l'une quelconque des revendications 6 à 9, dans lequel le ligand et le composé présentent la structure suivante :

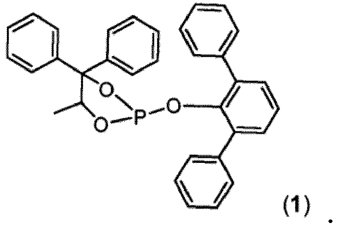

12. Procédé selon l'une quelconque des revendications 6 à 9, dans lequel le ligand et le composé présentent la structure suivante :

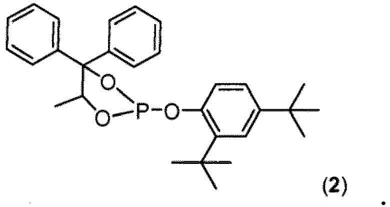

13. Procédé selon l'une quelconque des revendications 6 à 9, dans lequel le ligand et le composé présentent la structure suivante :

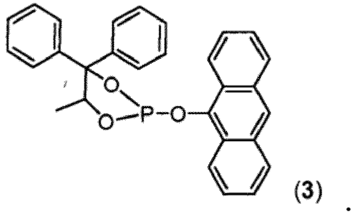

14. Procédé selon l'une quelconque des revendications 6 à 13, dans lequel le mélange réactionnel est porté à l'étape de procédé d) à une température dans la plage allant de 100 °C à 140 °C.
